# EUROPÄISCHE PATENTSCHRIFT

(11) **EP 0 745 587 B1**
(45) Veröffentlichungstag und Bekanntmachung des Hinweises auf die Patenterteilung: **09.12.1998**
(21) Anmeldenummer: 96108220.3
(22) Anmeldetag: 23.05.1996
(51) Int. Cl.: C07C 291/04

(54) **Verfahren zur Herstellung von Aminoxiden**
Process for the preparation of amine oxides
Procédé de préparation d'oxydes amines

(30) Priorität: 02.06.1995 DE 19520270
(43) Veröffentlichungstag der Anmeldung: 04.12.1996
(73) Patentinhaber: BASF AKTIENGESELLSCHAFT, 67056 Ludwigshafen (DE)
(72) Erfinder: Müller, Ulrich, Dr., 67434 Neustadt (DE); Massonne, Klemens, Dr., 67368 Westheim (DE); Eller, Karsten, Dr., 67059 Ludwigshafen (DE); Schulz, Michael, Dr., 67067 Ludwigshafen (DE)

(56) Entgegenhaltungen:
- EP-A- 0 092 862
- US-A- 4 565 891
- J. CHEM. SOC., CHEM. COMMUN., 1983, Seiten 1530-1532, XP002012281 D.P. RILEY: "Ruthenium Chloride Catalysed Oxidation of Tertiary Amines to Amine Oxides with Molecular Oxygen"

## Beschreibung

Die vorliegende Erfindung betrifft ein Verfahren zur Herstellung von Aminoxiden durch heterogenkatalytische Umsetzung von Aminen mit Wasserstoff/Sauerstoff-Mischungen an einem neuen Oxidationskatalysator auf Basis von Titan- oder Vanadiumsilikaten mit Zeolith-Struktur und einem Gehalt an Platinmetallen.

Die vorliegende Erfindung betrifft ein Verfahren zur Herstellung von Aminoxiden durch Umsetzung von Aminen mit Wasserstoff/Sauerstoffgemischen in Gegenwart von Oxidationskatalysatoren Elemente der VIII. Gruppe des Periodensystems auf Titansilikaten, Vanadiumsilikaten oder deren Gemische mit Zeolith-Struktur.

Die Herstellung von Trialkylaminoxiden durch Umsetzung der entsprechenden Amine mit Wasserstoffperoxid ist in Wasser als Lösungsmittel aus US-A-4 994 614, EP-A-320 694 und EP-A-426 084, in organischen Lösungsmitteln oder deren Gemischen aus EP-A-553 552, EP-A-401 503, US-A-5 055 233, US-A-5 082 600, US-A-5 130 488 und EP-A-307 184 bekannt. Aus DE-A-43 06 609 ist die polymeranaloge Umsetzung von Polyvinylpyridin zu Polyvinylpyridin-N-oxiden bekannt.

Nachteilig bei dem beschriebenen Verfahren ist die Verwendung von Wasserstoffperoxid als teurem Einsatzstoff.

Der vorliegenden Erfindung lag daher die Aufgabe zugrunde, den zuvor genannten Nachteilen abzuhelfen, insbesondere einen Weg zur Herstellung von Trialkylaminoxiden zu finden, ohne teures Wasserstoffperoxid einsetzen zu müssen.

Demgemäß wurde ein neues und verbessertes Verfahren zur Herstellung von Aminoxiden der allgemeinen Formel I 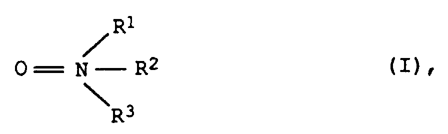 in der
- R¹,R²,R³: C₁- bis C₃₀-Alkyl, C₃- bis C₁₂-Cycloalkyl, C₁- bis C₃₀-Hydroxyalkyl, C₁- bis C₃₀-Aminoalkyl, C₂- bis C₃₀-Alkoxyalkyl, Aryl, C₇- bis C₂₀-Aralkyl, C₇- bis C₂₀-Alkylaryl, durch C₁- bis C₈-Alkyl, C₁- bis C₈-Alkoxy, Halogen ein- bis fünffach substituiertes C₃- bis C₁₂-Cycloalkyl, Aryl, C₇- bis C₂₀-Aralkyl, C₇- bis C₂₀-Alkylaryl, -[(CH₂)₂-O]ₙ-R⁴, -{[CH(CH₃)CH₂]₂-O}ₘ-R⁵, oder R¹ und R² gemeinsam eine gegebenenfalls durch Sauerstoff, Stickstoff oder Schwefel unterbrochene C₃-bie C₁₂-Alkylendikette
- R⁴,R⁵: C₁- bis C₁₂-Alkyl, C₃- bis C₁₂-Cycloalkyl, Hydroxy, -COR⁶ oder -CH₂-COOR⁷ C₁₂-Cycloalkyl, Aryl, C₇- bis C₂₀-Aralkyl, C₇- bis C₂₀-Alkylaryl,
- R⁶, R⁷: C₁- bis C₁₂-Alkyl oder C₃- bis C₁₂-Cycloalkyl und
- m, n: 1 bis 40
bedeuten, aus Aminen der allgemeinen Formel II 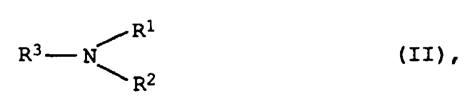 in der R¹, R² und R³,die oben genannten Bedeutungen haben, mit Wasserstoff/Sauerstoffgemischen bei Temperaturen von (-5) bis 90°C und Drücken von 1 bis 100 bar in Gegenwart von Oxidationskatalysatoren gefunden, welches dadurch gekennzeichnet ist, daß man als Oxidationskatalysatoren solche der VIII. Gruppe des Periodensystems der Elemente auf Titansilikaten, Vanadiumsilikaten oder deren Gemische mit Zeolith-Struktur einsetzt.

Das erfindungsgemäße Verfahren kann wie folgt durchgeführt werden:

Man kann die Amine II ohne oder bevorzugt in einem inerten Lösungsmittel und den Oxidationskatalysator, bevorzugt einen Heterogenkatalysator, bevorzugt in einem Druckapparat, beispielsweise einem Druckgefäß wie einem Autoklaven, einer Schüttelbombe und einem Festbettreaktor, besonders bevorzugt in einem Autoklaven oder einem druckstabilen Stahlreaktor vorlegen und mit einem Wasserstoff/Sauerstoffgemisch bei Temperaturen von (-5) bis 90°C, bevorzugt 15 bis 80°C, besonders bevorzugt 20 bis 75°C und Drücken (Gesamtdrücken) von 1 bis 100 bar, bevorzugt 5 bis 80 bar, besonders bevorzugt 10 bis 70 bar umsetzen.

Abhängig vom umzusetzenden Amin II kann die erfindungsgemäße Oxidation in flüssiger Phase, in der Gasphase oder auch in überkritischer Phase durchgeführt werden. Dabei wird der Katalysator bei Flüssigkeiten vorzugsweise als Suspension eingesetzt, während bei Gasphasen- oder überkritischer Fahrweise eine Festbettanordnung von Vorteil ist.

Wird die Oxidation in flüssiger Phase vorgenommen, arbeitet man in der Regel bei einem Druck von 1 bis 50 bar, vorteilhafterweise bei 1 bis 10 bar und in einer Suspensionsfahrweise in Gegenwart von Lösungsmitteln. Als Lösungsmittel eignen sich Alkohole, z.B. Methanol, Ethanol, iso-Propanol oder tert.-Butanol, Ketone wie z.B. Aceton, Diethylketon, Methylisobutylketon oder Nitrile wie Acetonitril oder Mischungen hieraus und insbesondere Wasser. Man kann auch Mischungen der genannten Lösungsmittel mit Wasser einsetzen. In bestimmten Fällen bewirkt die Verwendung von Wasser oder wasserhaltigen Lösungsmittelsystemen eine deutliche Selektivitätssteigerung des gewünschten Aminoxids gegenüber den reinen Alkoholen als Lösungsmittel.

Das Molverhältnis von Sauerstoff zu Wasserstoff kann üblicherweise im Bereich O₂:H₂ = 50:1 bis 1:1, bevorzugt 10:1 bis 1:1 liegen. Das Molverhältnis von Amin zum Sauerstoff liegt in der Regel bei 1:1 bis 3:1, vorzugsweise 1,5:1 bis 1,7:1. Als Trägergas kann ein beliebiges Inertgas zugefahren werden, insbesondere eignet sich Stickstoff.

Als Oxidationskatalysatoren eignen sich solche der VIII. Gruppe des Periodensystems der Elemente auf Titansilikaten, Vanadiumsilikaten oder deren Gemische mit Zeolith-Struktur, bevorzugt mit einem oder mehreren Elementen aus der Gruppe Eisen, Kobalt, Nickel, Ruthenium, Rhodium, Palladium, Osmium, Iridium und Platin, bevorzugt Ruthenium, Rhodium, Palladium, Osmium, Iridium und Platin, besonders bevorzugt Palladium, welche dadurch gekennzeichnet sind, daß die Platinmetalle jeweils in mindestens zwei verschiedenen Bindungsenergiezuständen vorliegen.

Der Gehalt an Elementen VIII. Gruppe des Periodensystems im erfindungsgemäßen Oxidationskatalysator beträgt 0,01 bis 30 Gew.-%, vorzugsweise 0,1 bis 15 Gew.-%, insbesondere 0,2 bis 5 Gew.-%, bezogen auf die Gesamtmasse des Oxidationskatalysators und somit beträgt der Anteil an Titansilikaten, Vanadiumsilikaten oder deren Gemische mit Zeolith-Struktur bevorzugt 99,99 bis 70 Gew.-%, besonders bevorzugt 99,9 bis 85 Gew.-%, insbesondere 99,8 bis 95 Gew.-%.

Außer mit den genannten Platinmetallen kann der erfindungsgemäße Oxidationskatalysator gegebenenfalls noch zusätzlich mit einem oder mehreren Elementen aus der Gruppe Rhenium, Silber und Gold modifiziert sein. Diese Elemente sind üblicherweise in einer Menge von 0,01 bis 10 Gew.-%, insbesondere 0,05 bis 5 Gew.-%, bezogen auf die Gesamtmasse des Oxidationskatalysators, enthalten.

Der Oxidationskatalysator sollte in der Regel bevorzugt vor seinem Einsatz in die speziellen Modifikation der Mischung aus verschiedenen Bindungsenergiezuständen überführt werden.

Die verschiedenen Bindungsenergiezustände entsprechen formell verschiedenen, also zwei oder mehreren Oxidationsstufen der Metalle. Es handelt sich hierbei in der Regel um verschiedene Bindungsenergiezustände. In einer bevorzugten Ausführungsform der erfindungsgemäßen Oxidationskatalysatoren liegen die Elemente der VIII. Gruppe des Periodensystems in zwei, drei, vier oder fünf, besonders bevorzugt in zwei, drei oder vier, insbesondere in zwei oder drei verschiedenen Bindungsenergiezuständen vor.

Beim Vorliegen von zwei verschiedenen Bindungsenergiezuständen kann dies beispielsweise eine Mischung aus Species der Oxidationsstufe 0 und (+1), 0 und (+2), 0 und (+3) oder 0 und (+4) sein. Die beiden Species liegen normalerweise im Verhältnis von 5:95 bis 95:5, insbesondere 10:90 bis 90:10 vor.

Beim Vorliegen von drei verschiedenen Bindungsenergiezuständen kann dies beispielsweise eine Mischung aus Species der Oxidationsstufe 0, (+1) und (+2) oder 0, (+2) und (+3) oder 0, (+2) und (+4) oder 0, (+1) und (+3) oder 0, (+1) und (+4) oder 0, (+3) und (+4) sein. Die drei Species liegen normalerweise im Verhältnis von (0,05 bis 20): (0,05 bis 20):1, insbesondere (0,1 bis 10): (0,1 bis 10):1 vor.

Es können weiterhin auch Mischungen aus vier oder mehr verschiedenen Oxidationsstufen vorliegen, beispielsweise aus 0, (+1), (+2) und (+3) oder 0, (+1), (+2) und (+4) oder 0, (+2), (+3) und (+4) oder 0, (+1), (+3) und (+4) oder 0, (+1), (+2), (+3) und (+4). Die Species liegen hierbei in ähnlichen Gewichtsverhältnissen zueinander wie bei den Mischungen aus 2 oder 3 verschiedenen Oxidationsstufen vor.

Unter den Platinmetallen wird Palladium bevorzugt. In einer besonders bevorzugten Ausführungsform liegt das Palladium in zwei oder drei verschiedenen Bindungsenergiezuständen vor.

Die Bindungsenergiezustände an der Oberfläche des Katalysators können am einfachsten durch Röntgenphotoelektronenspektroskopie (XPS) charakterisiert werden. So liegen bei einer typischen Mischung von drei Palladiumspecies die entsprechenden Werte für die Energien des Pd-3d5/2-Zustandes bei 335,0 bis 335,4 eV, 336 bis 336,6 eV und 337,1 bis 337,9 eV, was formell den Oxidationsstufen Pd⁰, Pd¹⁺ und Pd²⁺ entspricht.

Bei den erfindungsgemäßen Oxidationskatalysatoren ist es besonders vorteilhaft, die Platinmetalle derart aufzubringen, daß keine Metall-Metall-Bindungen wirksam werden und Metall-Zeolith-Bindungen überwiegen. Insbesondere aus Untersuchungen der Röntgenfeinstruktur (EXAFS) geht hervor, daß es beim Vorliegen von Palladium wesentlich ist, daß nahezu ausschließlich Palladium-Sauerstoff-Bindungsabstände von 2,02 + 0,02 Å auftreten und Palladium-Palladium-Abstände wie bei ausgedehntem Palladium-Metall oder Palladium-Agglomeraten von 2,74 + 0,02 Å sowie PalladiumPalladium-Abstände von 3,04 + 0,02 Å wie in Palladium-(II)-oxid vermieden werden.

Die Basis für den erfindungsgemäßen Oxidationskatalysator bilden bekannte Titan- oder Vanadiumsilikate mit Zeolith-Struktur, vorzugsweise mit Pentasil-Zeolith-Struktur, insbesondere die Typen mit röntgenographischer Zuordnung zur MFI-, MEL- oder MFI/MEL-Mischstruktur. Zeolithe dieses Typs sind beispielsweise in W.M. Meier und D.H. Olson, Atlas of Zeolite Structure Types, Butterworths, 2nd Ed., 1987, beschrieben. Denkbar sind weiterhin titanhaltige Zeolithe mit der Struktur des ZSM-48, Ferrierit, beta-Zeolith oder ZSM-12.

Im erfindungsgemäßen Oxidationskatalysator kann das Titan des Silikalits teilweise oder vollständig durch Vanadium ersetzt sein. Das molare Verhältnis von Titan und/oder Vanadium zur Summe aus Silicium plus Titan und/oder Vanadium liegt in der Regel im Bereich von 100:1 bis 5:1, bevorzugt 80:1 bis 10:1, besonders bevorzugt 60:1 bis 20:1.

Der erfindungsgemäße Oxidationskatalysator wird zweckmäßigerweise durch Imprägnieren oder Umsetzen von Titan- oder Vanadiumsilikaliten mit Zeolith-Struktur mit Salzlösungen, Chelatkomplexen oder Carbonylkomplexen der Platinmetalle hergestellt, wobei diese Herstellweise dadurch gekennzeichnet ist, daß man im Anschluß an die Imprägnierung bzw. Umsetzung durch geeignete reduzierende oder oxidierende Bedingungen die erforderliche Verteilung der Bindungsenergiezustände der Platinmetalle einstellt.

So kann das Aufbringen der Platinmetalle beispielsweise durch Imprägnieren mit einer Platinmetallsalzlösung, insbesondere in der Oxidationsstufe (+2) bis (+4), aus rein wäßriger, rein alkoholischer oder wäßrig-alkoholischer Mischung bei Temperaturen von 20 bis 90°C, insbesondere 30 bis 60°C erfolgen. Als Salze können dabei z.B. die entsprechenden Chloride, Acetate oder deren TetraminKomplexe eingesetzt werden, im Fall von Palladium sollen hier Palladium-(II)-halogenide, wie Chloride oder Bromide, Palladium-(II)-acetat und der Palladium-(II)-tetraminchloro-Komplex genannt werden. Hierbei ist die Menge der Metallsalze so zu wählen, daß auf dem resultierenden Oxidationskatalysator Konzentrationen von 0,01 bis 20 Gew.-% an Platinmetall erzielt werden.

Ebenso kommt hier die Umsetzung mit entsprechenden Chelatkomplexen der Platinmetalle in unpolaren Lösungsmitteln in Betracht, etwa mit Acetylacetonaten, Acetonylacetonaten oder Phosphinkomplexen.

Auch das Aufbringen in Form von entsprechenden Carbonylkomplexen der Platinmetalle ist möglich. Hierbei arbeitet man zweckmäßigerweise in der Gasphase unter erhöhtem Druck oder imprägniert mit diesen Carbonylkomplexen in überkritischen Lösungsmitteln wie CO₂.

Nach gegebenenfalls erforderlicher Trocknung und/oder gegebenenfalls einem Brennschritt der so erhaltenen Katalysatorvorstufe wird die Verteilung der Bindungsenergiezustände vorzugsweise durch partielle Reduktion vorliegender höherer Oxidationsstufen der Platinmetalle, insbesondere durch Hydrierung in einer Wasserstoffatmosphäre, eingestellt. Liegen die Platinmetalle bereits in der Oxidationsstufe 0 vor, so beim Aufbringen als Carbonylkomplexe, muß partiell oxidiert werden.

In einer bevorzugten Ausführungsform wird der erfindungsgemäße Oxidationskatalysator mit Salzlösungen der Platinmetalle in der Oxidationsstufe (+2) bis (+4) imprägniert und anschließend der getrocknete Katalysator in einer Wasserstoffatmosphäre hydriert, wobei diese Herstellweise dadurch gekennzeichnet ist, daß man die Hydrierung bei Temperaturen von 20 bis 120°C, bevorzugt 25 bis 100°C, besonders bevorzugt 30 bis 70°C durchführt.

Wird bei dieser partiellen Reduktion durch Hydrierung in einer Wasserstoffatmosphäre die Temperatur zu hoch gewählt, liegen die Platinmetalle nahezu ausschließlich in der Oxidationsstufe 0, d.h. als Metalle, und in Form größerer Agglomerate vor, was im mikroskopischem Bild am Auftreten von Metall-Clustern mit Größen über 1 nm erkennbar ist.

Die vorgenannten Titan- oder Vanadiumsilikalite mit Zeolith-Struktur, insbesondere hierbei solche mit MFI-Pentasil-Zeolith-Struktur, werden in der Regel hergestellt, indem man ein Synthesegel, bestehend aus Wasser, einer Titan- bzw. Vanadiumquelle und Siliciumdioxid in geeigneter Weise unter Zusatz von organischen stickstoffhaltigen Verbindungen (Schablonen-Verbindungen) unter hydrothermalen Bedingungen und gegebenenfalls unter Zusatz von Ammoniak, Alkali oder Fluorid als Mineralisatoren kristallisiert. Als organische stickstoffhaltige Verbindungen kommen beispielsweise 1,6-Diaminohexan oder Salze oder das freie Hydroxid von Tetraalkylammonium, speziell von Tetrapropylammonium, in Betracht.

Bei der Herstellung der Titan- bzw. Vanadiumsilikalite muß eine Verunreinigung mit größeren Mengen an Alkali- oder Erdalkalimetallverbindungen vermieden werden; Alkaligehalte (insbesondere an Natrium oder Kalium) < 100 ppm sind erstrebenswert, um später einen ausreichend aktiven Oxidationskatalysator zu erhalten. Die Kristallisation der phasenreinen Struktur des Titan- bzw. Vanadiumsilikalits erfolgt vorzugsweise bei Temperaturen von 140 bis 190°C, insbesondere 160 bis 180°C, innerhalb einer Zeitdauer von 2 bis 7 Tagen, wobei bereits nach ca. 4 Tagen gut kristallines Produkt erhalten wird. Durch starkes Rühren und einen hohen pH-Wert von 12 bis 14 während der Kristallisation kann die Synthesedauer einerseits und die Kristallitgröße andererseits deutlich verringert werden.

Von Vorteil sind beispielsweise Primärkristallite von 0,05 bis 0,5 µm, insbesondere aber solche mit Größen von weniger als 0,2 µm im mittleren Partikeldurchmesser.

Nach der Kristallisation kann der Titan- bzw. Vanadiumsilikalit nach an sich bekannten Methoden abfiltriert, gewaschen und bei 100 bis 120°C getrocknet werden.

Zur Entfernung der in den Poren noch vorliegenden Amin- oder Tetraalkylammoniumverbindungen kann das Material noch einer thermischen Behandlung an Luft oder unter Stickstoff unterzogen werden. Dabei ist es vorteilhaft, das Abbrennen des Templates unter Bedingungen vorzunehmen, die den Temperaturanstieg auf Werte < 550°C begrenzen.

Zur Modifizierung des erfindungsgemäßen Oxidationskatalysators können außer den schon genannten Zusätzen von Platinmetallen und 5 sonstigen Elementen die nach dem derzeitigen Stand der Technik bekannten Methoden der Verformung unter Zuhilfenahme eines Binders, des Ionenaustausches und der Oberflächenmodifizierung, beispielsweise über chemical vapor deposition (CVD) oder chemische Derivatisierung wie etwa Silylierung, zum Einsatz gelangen. Das Vorliegen der für eine Oxidationsreaktion benötigen Katalysatorfunktionen kann durch IR-Spektroskopie geprüft werden: bei 550 cm⁻¹ und bei 960 cm⁻¹ treten signifikante Banden auf, die das Vorliegen der erwünschten Festkörper-Kristallinität sowie der benötigten Oxidationsaktivität anzeigen.

Eine Regenerierung der erfindungsgemäßen Oxidationskatalysatoren ist ebenfalls möglich. Desaktivierte Katalysatoren können durch kontrolliertes Abbrennen von Kohlenstoffbelegungen im Temperaturbereich von 350 bis 650°C und nachfolgende Reduktion beispielsweise mit Wasserstoff wieder in eine aktive Form zurückgeführt werden.

Bei geringer Belegung kann der Katalysator auch durch einen einfachen Waschprozeß wieder regeneriert werden. Je nach Bedarf kann der Waschvorgang im neutralen, sauren oder basischen pH-Bereich durchgeführt werden. Gegebenenfalls kann auch mittels einer mineralsauren Wasserstoffperoxidlösung die Katalysatoraktivität wieder regeneriert werden.

Die Substituenten R¹, R², R³, R⁴, R⁵, R⁶, R⁷ und die Indizes m und n in den Verbindungen I und II haben folgende Bedeutungen:
R¹, R², R³, R⁴, R⁵, R⁶, R⁷
   - C₃- bis C₁₂-Cycloalkyl, bevorzugt C₃- bis C₈-Cycloalkyl wie Cyclopropyl, Cyclobutyl, Cyclopentyl, Cyclohexyl, Cycloheptyl und Cyclooctyl, besonders bevorzugt Cyclopentyl, Cyclohexyl und Cyclooctyl,
R¹, R², R³, R⁴, R⁵,
   - Aryl wie Phenyl, 1-Naphthyl, 2-Naphthyl, 1-Anthryl, 2-Anthryl und 9-Anthryl, bevorzugt Phenyl, 1-Naphthyl und 2-Naphthyl, besonders bevorzugt Phenyl,
   - C₇- bis C₂₀-Aralkyl, bevorzugt C₇- bis C₁₂-Phenylalkyl wie Benzyl, 1-Phenethyl, 2-Phenethyl, 1-Phenyl-propyl, 2-Phenylpropyl, 3-Phenyl-propyl, 1-Phenyl-butyl, 2-Phenyl-butyl, 3-Phenyl-butyl und 4-Phenyl-butyl, besonders bevorzugt Benzyl, 1-Phenethyl und 2-Phenethyl,
   - C₇- bis C₂₀-Alkylaryl, bevorzugt C₇- bis C₁₂-Alkylphenyl wie 2-Methylphenyl, 3-Methylphenyl, 4-Methylphenyl, 2,4-Dimethylphenyl, 3,5-Dimethylphenyl, 2,6-Dimethylphenyl, 3, 4-Dimethylphenyl, 3,5-Dimethylphenyl, 2,3,4-Trimethylphenyl, 2,3,5-Trimethylphenyl, 2,3,6-Trimethylphenyl, 2,4,6-Trimethylphenyl, 2-Ethylphenyl, 3-Ethylphenyl, 4-Ethylphenyl, 2-n-Propylphenyl, 3-n-Propylphenyl und 4-n-Propylphenyl,
R¹,R²,R³
   - C₁- bis C₃₀-Alkyl, bevorzugt C₁- bis C₂₀-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, n-Nonyl, iso-Nonyl, n-Decyl, iso-Decyl, n-Undecyl, iso-Undecyl, n-Dodecyl, iso-Dodecyl, n-Tetradecyl, n-Hexadecyl und n-Octadecyl, besonders bevorzugt Methyl, Ethyl, n-Propyl, iso-Propyl, n-Butyl, iso-Butyl, sec.-Butyl, tert.-Butyl, n-Octyl, n-Decyl, n-Dodecyl, n-Tetradecyl, n-Hexadecyl und n-Octadecyl,
   - C₁- bis C₃₀-Hydroxyalkyl, bevorzugt C₁- bis C₈-Hydroxyalkyl, besonders bevorzugt C₁- bis C₄-Hydroxyalkyl wie Hydroxymethyl, 1-Hydroxyethyl, 2-Hydroxyethyl, 1-Hydroxy-n-propyl, 2-Hydroxy-n-propyl, 3-Hydroxy-n-propyl und 1-Hydroxy-methylethyl,
   - C₁- bis C₃₀-Aminoalkyl, bevorzugt C₁- bis C₂₀-Aminoalkyl, besonders bevorzugt C₁- bis C₈-Aminoalkyl wie Aminomethyl, 1-Aminoethyl, 2-Aminoethyl, 1-Amino-n-propyl, 2-Amino-n-propyl, 3-Amino-n-propyl und 1-Aminomethyl-ethyl,
   - C₂- bis C₃₀-Alkoxyalkyl, bevorzugt C₂- bis C₂₀-Alkoxyalkyl, besonders bevorzugt C₂- bis C₈-Alkoxyalkyl wie Methoxymethyl, Ethoxymethyl, n-Propoxymethyl, iso-Propoxymethyl, n-Butoxymethyl, iso-Butoxymethyl, sec.-Butoxymethyl, tert.-Butoxymethyl, 1-Methoxyethyl und 2-Methoxyethyl,
   - durch C₁- bis C₈-Alkyl, C₁- bis C₈-Alkoxy, Halogen ein- bis fünffach substituiertes C₃- bis C₁₂-Cycloalkyl, bevorzugt durch C₁- bis C₄-Alkyl, C₁- bis C₄-Alkoxy, Halogen ein- bis dreifach substituiertes C₃- bis C₈-Cycloalkyl, besonders bevorzugt durch C₁- bis C₂-Alkyl, C₁- bis C₂-Alkoxy, Halogen ein- bis dreifach substituiertes C₅- bis C₈-Cycloalkyl,
   - durch C₁- bis C₈-Alkyl, C₁- bis C₈-Alkoxy, Halogen ein- bis fünffach substituiertes Aryl, bevorzugt durch C₁- bis C₄-Alkyl, C₁- bis C₄-Alkoxy, Halogen ein- bis dreifach substituiertes Phenyl, besonders bevorzugt durch C₁- bis C₂-Alkyl, C₁- bis C₂-Alkoxy, Halogen ein- bis dreifach substituiertes Phenyl,
   - durch C₁- bis C₈-Alkyl, C₁- bis C₈-Alkoxy, Halogen ein- bis fünffach substituiertes C₇- bis C₂₀-Aralkyl, bevorzugt durch C₁- bis C₄-Alkyl, C₁- bis C₄-Alkoxy, Halogen ein- bis dreifach substituiertes C₇- bis C₁₆-Phenalkyl, besonders bevorzugt durch C₁- bis C₂-Alkyl, C₁- bis C₂-Alkoxy, Halogen ein- bis dreifach substituiertes C₇- bis C₁₂-Phenalkyl,
   - durch C₁- bis C₈-Alkyl, C₁- bis C₈-Alkoxy, Halogen ein- bis fünffach substituiertes C₇- bis C₂₀-Alkylaryl, bevorzugt durch C₁- bis C₄-Alkyl, C₁- bis C₄-Alkoxy, Halogen ein- bis dreifach substituiertes C₇- bis C₁₆-Alkylphenyl, besonders bevorzugt durch C₁- bis C₂-Alkyl, C₁- bis C₂-Alkoxy, Halogen ein- bis dreifach substituiertes C₇- bis C₁₂-Alkylphenyl,
   - -[(CH₂)₂-O]ₙ-R⁴,
   - -{[CH(CH₃)CH₂]-O}ₘ-R⁵,
R¹ und R² gemeinsam
   - eine C₃- bie C₁₂-Alkylendikette wie -(CH₂)₃-, -[(CH₃)CH-CH₂]-, -(CH₂)₄-, -[CH₂-(CH₃)CH-CH₂]-, -[(CH₃)CH-(CH₂)₂]-, -(CH₂)₅-, -(CH₂)₆-, -(CH₂)₇-, -(CH₂)₈-, -(CH₂)₉-, -(CH₂)₁₀-, -(CH₂)₁₁- und -(CH₂)₁₂-, bevorzugt eine C₃- bie C₈-Alkylendikette wie -(CH₂)₃-, -[(CH₃)CH-CH₂]-, - (CH₂)₄-, -[CH₂-(CH₃)CH-CH₂]-, -[(CH₃)CH-(CH₂)₂]-, -(CH₂)₅-, -(CH₂)₆-, -(CH₂)₇- und -(CH₂)₈-,
   - eine durch Sauerstoff, Stickstoff oder Schwefel unterbrochene C₃- bie C₁₂-Alkylendikette, bevorzugt eine durch Sauerstoff oder Stickstoff unterbrochene C₃- bie C₈-Alkylendikette, besonders bevorzugt eine durch Sauerstoff oder Stickstoff unterbrochene C₄- bie C₆-Alkylendikette -[(CH₂)₂-N-(CH₂)₂]-, -[(CH₂)₂-O-(CH₂)₂]-, -[(CH₂)₃-N-(CH₂)₂]-, -[(CH₂)₃-O-(CH₂)₂]-, -[(CH₂)₄-N-(CH₂)₂]-, -[(CH₂)₄-O-(CH₂)₂]-, -[(CH₂)₃-N-(CH₂)₃]- und -[(CH₂)₃-O-(CH₂)₃]-,
R⁴,R⁵
   - Hydroxy,
   - -COR⁶ oder
   - -CH₂-COOR⁷,
R⁴,R⁵,R⁶,R⁷
   - C₁- bis C₁₂-Alkyl, bevorzugt C₁- bis C₈-Alkyl wie Methyl, Ethyl, n-Propyl, iso-Propyl, n-Butyl, iso-Butyl, sec.-Butyl, tert.-Butyl, n-Pentyl, iso-Pentyl, sec.-Pentyl, neo-Pentyl, 1,2-Dimethylpropyl, n-Hexyl, iso-Hexyl, sec.-Hexyl, n-Heptyl, iso-Heptyl, n-Octyl, iso-Octyl, besonders bevorzugt C₁- bis C₄-Alkyl wie Methyl, Ethyl, n-Propyl, iso-Propyl, n-Butyl, iso-Butyl, sec.-Butyl und tert.-Butyl und
m, n
   - eine ganze Zahl von 1 bis 40, bevorzugt 2 bis 20 wie 2, 3, 4, 5, 6, 7, 8, 9, 10, 11, 12, 13, 14, 15, 16, 17, 18, 19 und 20, besonders bevorzugt 3 bis 15 wie 3, 4, 5, 6, 7, 8, 9, 10, 11, 12, 13, 14 und 15.

Ebenso können in Art einer polymeranalogen Reaktion z.B. Polyalkylenamine zu den entsprechenden Polyalkylenamin-N-Oxiden umgesetzt werden. Dies gilt ebenfalls für polymere Pyridine, wie etwa bei der Umsetzung von Polyvinylpyridin zu Polyvinylpyridin-N-Oxid.

Typische Beispiele für die Amine II sind Dimethyloctylamin, Diethyloctylamin, Dimethylbutylamin, Diethylhexylamin, Dimethylisobutylamin, Diethyl-2-ethylhexylamin, Dimethyldecylamin, Dimethyldodecylamin, Dimethyltetradecylamin, Dimethylhexadecylamin, Dimethyloctadecylamin, Tributylamin, Trimethylamin, Butyldiethylamin, Triethanolamin, Dimethylethanolamin, Methyldiethanolamin, Dibenzylbutylamin, Dicyclohexylmethylamin, N-Methylmorpholin, N-Butylmorpholin, N-Methylpiperidin, N-Butylpyrrolidin.

Bevorzugt sind solche Amine II, die zwei kurzkettige Alkylreste wie Methyl und Ethyl neben einem langkettigen Rest mit 6 bis 20 C-Atomen enthalten wie z.B. Dimethyloctylamin, Dimethyldecylamin, Dimethyldodecylamin, Dimethyltetradecylamin, Dimethyloctadecylamin oder Amine, deren Stickstoffatom Teil eines heterocyclischen Ringes ist wie Butylpiperidin oder Propylpyrrolidin oder Methylmorpholin oder Gemische davon.

Das erfindungsgemäße Verfahren eignet sich in besonderem Maße für Oxidierung von N-Methylmorpholin zu N-Methylmorpholinoxid.

Tertiäre Aminoxide finden weite Anwendung als Chemikalien und Zwischenprodukte (Kirk, Othmer, 4. Ausgabe Vol. 2 Seite 357 ff.). So sind die Oxide von Aminen mit langer Alkylkette, wie z.B. Dimethyldodecylamin, Bestandteile waschaktiver Formulierungen. N-Methylmorpholin-N-oxid ist ein gut geeignetes Lösemittel für Cellulose zur Herstellung von Cellulosefasern (vgl. z.B. DE-A-16 94 048). Desweiteren wird es bei der Dihydroxylierung von Olefinen als Oxidationsmittel verwendet [J. Am. Chem. Soc. 98, 1986 bis 1987 (1976)] und findet so bei der Zwischenproduktherstellung z.B. für Pharmazeutika Verwendung.

### Beispiele

### Katalysator A

### Kristallisation eines Titansilikalits

Zu 455 g Tetraethylorthosilikat wurden innerhalb von 30 min 15 g Tetraisopropylorthotitanat, anschließend 800 g einer 20 Gew.-%igen wäßrigen Tetrapropylammoniumhydroxid-Lösung (Alkaligehalt < 10 ppm) zugegeben, 1 Stunde nachgerührt und bei 90 bis 100°C abdestilliert. Dem Sumpf wurden 1,5 Liter deionisiertes Wasser zugesetzt und in einem Rührautoklaven 92 Stunden bei einer Temperatur von 175°C gehalten. Das erkaltete Reaktionsgemisch wurde abzentrifugiert, mit Wasser neutralgewaschen, bei 110°C innerhalb von 24 Stunden getrocknet (Auswaage 149 g) und unter Luft bei 500°C in 5 Stunden das im Zeolithen noch vorhandene Templat abgebrannt (Kalzinierungsverlust: 14 Gew.-%).

Das Produkt hatte nach naßchemischer Analyse einen Ti-Gehalt von 1,5 Gew.-% und einen Restgehalt an Alkali (Kalium) unterhalb von < 0,01 Gew.-%. Die Ausbeute (auf eingesetztes SiO₂ gerechnet) betrug 97%. Die Kristallitgröße lag bei ca. 0,1 bis 0,15 µm und das Produkt zeigte im IR typische Banden bei 960 cm⁻¹ und 550 cm⁻¹.

### Katalysator B

### Aufbringung der Aktivkomponente

Eine Lösung aus 0,515 g Palladium(II)-chlorid und 120 g Ammoniaklösung (25 Gew.-% in Wasser) wurde zu 60 g des frisch hergestellten Titansilikalits aus Katalysator A in 130 g deionisiertem Wasser gegeben, 1 Stunde gerührt und bei 90 bis 100°C/5 mbar eingedampft. Das Produkt wurde direkt zur Reduktion eingesetzt.

### Aktivierung des Katalysators B

In einem Drehrohrofen (Quarzglas, Durchmesser 5 cm, Länge in der Heizzone 20 cm) wurden 20 g des Pd-imprägnierten Produktes innerhalb von 90 min bei einer Temperatur von 50°C mit einer Gasmischung aus 20 l/h Stickstoff und 1 l/h Wasserstoff bei einer Drehzahl des Ofens von 50 U/min reduziert. Das fertige Produkt zeigte mittels transmissionselektronenmikroskopischer (TEM)-Analyse keine metallischen Palladium-Cluster mit Größen über 1,0 nm. Der Palladiumgehalt wurde naßchemisch zu 0,49 Gew.-% bestimmt. Mittels XPS fand man die drei vorn genannten Bindungsenergiezustände des Pd-3d5/2-Photoelektrons [formell entsprechend den Oxidationsstufen (+2), (+1) und 0]. EXAFS-Messungen an dieser Probe zeigten ein Signal für Pd-O-oder Pd-N-Bindungsabstände von 2,02 + 0,02 Å. Pd-Pd-Bindungsabstände von 2,74 + 0,02 Å oder 3,04 + 0,02 Å wurden nicht beobachtet.

### Beispiel 1

In einem Autoklav wurden 3,66 g N-Methylmorpholin, 141,3 ml dest. Wasser und 2 g Katalysator B bei Raumtemperatur und 20 bar Sauerstoff und 20 bar Wasserstoff 2 Stunden bei 30°C gerührt. Nach Entspannung, Abzentrifugieren des Katalysators und Waschen des Katalysators wurde der Gehalt an N-Methylmorpholin und N-Methylmorpholinoxid in Austrags- und Waschlösung durch Titration mit 1 N Salzsäure bestimmt. Die Ergebnisse sind in Tabelle 1 zusammengestellt.

**Tabelle 1**

| | Masse [g] | N-Methylmorpholin [Gew.-%] | N-Methylmorpholinoxid [Gew.-%] |
|---|---|---|---|
| Produktlösung | 147,3 | 2,03 | 0,3 |
| Waschlösung | 98,1 | 0,07 | 0 |

| | |
|---|---|
| N-Methylmorpholin-Umsatz | 16,5% |
| Selektivität | 62% |
| N-Methylmorpholinoxid-Ausbeute | 10,4% |

### Beispiel 2

Analog Beispiel 1 wurden 5 g N-Methylmorpholin bei einer Reaktionstemperatur von 60°C und einer Reaktionszeit von 5 Stunden umgesetzt. Die Ergebnisse sind in Tabelle 2 zusammengestellt.

**Tabelle 2**

| | Masse [g] | N-Methylmorpholin [Gew.-%] | N-Methylmorpholinoxid [Gew.-%] |
|---|---|---|---|
| Produktlösung | 148,1 | 0 | 2,7 |
| Waschlösung | 57,5 | 0,03 | 0 |

| | |
|---|---|
| N-Methylmorpholin-Umsatz | 98% |
| Selektivität | 73% |
| N-Methylmorpholinoxid-Ausbeute | 72% |

### Beispiel 3

In einem Autoklav wurden 144 ml dest. Wasser, 15 g N-Methylmorpholin und 6 g Katalysator B bei Raumtemperatur und 20 bar Sauerstoff und 20 bar Wasserstoff 5 Stunden bei 30°C gerührt. Nach Entspannung, Abzentrifugieren des Katalysators und Waschen des Katalysators wurde der Gehalt an N-Methylmorpholin und N-Methylmorpholinoxid in Austrags- und Waschlösung durch Titration mit 1 N Salzsäure bestimmt. Die Ergebnisse sind in Tabelle 3 zusammengestellt.

**Tabelle 3**

| | Masse [g] | N-Methylmorpholin [Gew.-%] | N-Methylmorpholinoxid [Gew.-%] |
|---|---|---|---|
| Produktlösung | 148,5 | 2,5 | 4,53 |
| Waschlösung | 67,5 | 0,17 | 0,34 |

| | |
|---|---|
| N-Methylmorpholin-Umsatz | 74% |
| Selektivität | 54% |
| N-Methylmorpholinoxid-Ausbeute | 40% |

### Beispiel 4

Analog Beispiel 3 wurden 6,5 g Katalysator B eingesetzt. Die Reaktionszeit betrug 8 Stunden. Die Ergebnisse sind in Tabelle 4 zusammengestellt.

**Tabelle 4**

| | Masse [g] | N-Methylmorpholin [Gew.-%] | N-Methylmorpholinoxid [Gew.-%] |
|---|---|---|---|
| Produktlösung | 145,5 | 1,2 | 5,63 |
| Waschlösung | 62,2 | 0,17 | 0,66 |

| | |
|---|---|
| N-Methylmorpholin-Umsatz | 88% |
| Selektivität | 57% |
| N-Methylmorpholinoxid-Ausbeute | 50% |

### Vergleichsbeispiel I

In einem Autoklav wurden 144 ml dest. Wasser, 11 g N-Methylmorpholin und 4,5 g (5% Pd auf Kohle) vorgelegt, bei Raumtemperatur nacheinander 20 bar Sauerstoff und 20 bar Wasserstoff aufgepreßt und 8 Stunden bei 60°C gerührt. Nach Abkühlung und Entspannung wurde der Katalysator abzentrifugiert, mit Wasser gewaschen.

Austrag und Waschlösung mit 1 N Salzsäure titriert. Die Ergebnisse sind in Tabelle I zusammengestellt.

**Tabelle I**

| | Masse [g] | N-Methylmorpholin [Gew.-%] | N-Methylmorpholinoxid [Gew.-%] |
|---|---|---|---|
| Produktlösung | 136,9 | 7,7 | 0 |
| Waschlösung | 80,3 | 0,49 | 0 |

### Vergleichsbeispiel II

Analog Beispiel I wurden 5 g N-Methylmorpholin und 2 g Katalysator A (Titansilikalit) bei einer Reaktionstemperatur von 30°C und einer Reaktionszeit von 2 Stunden umgesetzt. eingesetzt. Die Ergebnisse sind in Tabelle II zusammengestellt.

**Tabelle II**

| | Masse [g] | N-Methylmorpholin [Gew.-%] | N-Methylmorpholinoxid [Gew.-%] |
|---|---|---|---|
| Produktlösung | 148 | 2,91 | 0 |
| Waschlösung | 53,7 | < 0,1 | 0 |

## Patentansprüche

1. Verfahren zur Herstellung von Aminoxiden der allgemeinen Formel I 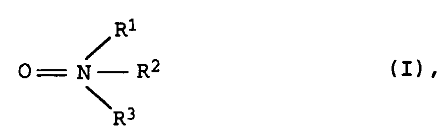 in der
R¹,R²,R³ C₁- bis C₃₀-Alkyl, C₃- bis C₁₂-Cycloalkyl, C₁- bis C₃₀-Hydroxyalkyl, C₁- bis C₃₀-Aminoalkyl, C₂- bis C₃₀-Alkoxyalkyl, Aryl, C₇- bis C₂₀-Aralkyl, C₇- bis C₂₀-Alkylaryl, durch C₁- bis C₈-Alkyl, C₁- bis C₈-Alkoxy, Halogen ein- bis fünffach substituiertes C₃- bis C₁₂-Cycloalkyl, Aryl, C₇- bis C₂₀-Aralkyl, C₇- bis C₂₀-Alkylaryl, -[(CH₂)₂-O]n-R⁴, -{[CH(CH₃)CH₂]-O}ₘ-R⁵, oder R¹ und R² gemeinsam eine gegebenenfalls durch Sauerstoff, Stickstoff oder Schwefel unterbrochene C₃- bis C₁₂-Alkylendikette
R⁴,R⁵ C₁- bis C₁₂-Alkyl, C₃- bis C₁₂-Cycloalkyl, Hydroxy, -COR⁶ oder -CH₂-COOR⁷, C₃- bis C₁₂-Cycloalkyl, Aryl, C₇- bis C₂₀-Aralkyl, C₇- bis C₂₀-Alkylaryl,
R⁶,R⁷ C₁- bis C₁₂-Alkyl oder C₃- bis C₁₂-Cycloalkyl und
m, n 1 bis 40
bedeuten, aus Aminen der allgemeinen Formel II 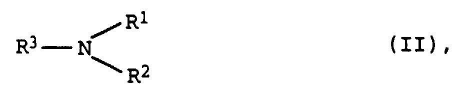 in der R¹, R² und R³,die oben genannten Bedeutungen haben, mit Wasserstoff/Sauerstoffgemischen bei Temperaturen von (-5) bis 90°C und Drücken von 1 bis 100 bar in Gegenwart von Oxidationskatalysatoren, dadurch gekennzeichnet, daß man als Oxidationskatalysatoren solche der VIII. Gruppe des Periodensystems der Elemente auf Titansilikaten, Vanadiumsilikaten oder deren Gemische mit Zeolith-Struktur einsetzt.

2. Verfahren zur Herstellung von Aminoxiden I nach Anspruch 1, dadurch gekennzeichnet, daß die Oxidationskatalysatoren 0,01 bis 20 Gew.-% der Elemente der VIII. Gruppe des Periodensystems der Elemente enthalten.

3. Verfahren zur Herstellung von Aminoxiden I nach den Ansprüchen 1 bis 2, dadurch gekennzeichnet, daß die Oxidationskatalysatoren als Elemente der VIII. Gruppe des Periodensystems der Elemente Ruthenium, Rhodium, Palladium, Osmium, Iridium, Platin oder deren Gemische enthalten.

4. Verfahren zur Herstellung von Aminoxiden I nach den Ansprüchen 1 bis 3, dadurch gekennzeichnet, daß die Oxidationskatalysatoren zusätzlichen ein oder mehrere Elemente aus der Gruppe Rhenium, Silber und Gold enthalten.

5. Verfahren zur Herstellung von Aminoxiden I nach den Ansprüchen 1 bis 3, dadurch gekennzeichnet, daß die Oxidationskatalysatoren aus 0,01 bis 20 Gew.-% der Elemente der VIII. Gruppe des Periodensystems der Elemente und 99,99 bis 80 Gew.-% Titansilikaten, Vanadiumsilikaten oder deren Gemische mit Zeolith-Struktur bestehen.

6. Verfahren zur Herstellung von Aminoxiden I nach den Ansprüchen 1 bis 5, dadurch gekennzeichnet, daß die Elemente der VIII. Gruppe des Periodensystems der Elemente in mindestens zwei verschiedenen Bindungsenergiezuständen vorliegen.

7. Verfahren zur Herstellung von Aminoxiden I nach den Ansprüchen 1 bis 6, dadurch gekennzeichnet, daß die Oxidationskatalysatoren als Elemente der VIII. Gruppe des Periodensystems der Elemente Palladium, das in zwei oder drei verschiedenen Bindungsenergiezuständen vorliegt, enthalten.

8. Verfahren zur Herstellung von Aminoxiden I nach den Ansprüchen 1 bis 7, dadurch gekennzeichnet, daß das Molverhältnis von Silicium zum Titan, Vanadium oder deren Gemische im Oxidationskatalysator 100:1 bis 5:1 beträgt.

9. Verfahren zur Herstellung von Aminoxiden I nach den Ansprüchen 1 bis 8, dadurch gekennzeichnet, daß R¹,R²,R³ C₁-bis C₄-Alkyl, R¹ und R² gemeinsam-(CH₂)₂-O-(CH₂)₂- oder -(CH₂)₂-NR¹-(CH₂)₂-, R³ zusätzlich n-Decyl, n-Dodecyl, n-Tetradecyl, n-Hexadecyl und n-Octadecyl bedeuten.

10. Verfahren zur Herstellung von Aminoxiden I nach den Ansprüchen 1 bis 9, dadurch gekennzeichnet, daß R¹ und R² gemeinsam -(CH₂)₂-O-(CH₂)₂- und R³ Methyl bedeuten.

## Claims

1. A process for preparing amine oxides of the general formula I 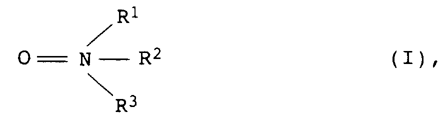 where
R¹, R² and R³ are each C₁-C₃₀-alkyl, C₃-C₁₂-cycloalkyl, C₁-C₃₀-hydroxyalkyl, C₁-C₃₀-aminoalkyl, C₂-C₃₀-alkoxyalkyl, aryl, C₇-C₂₀-aralkyl, C₇-C₂₀-alkylaryl, C₁-C₈-alkyl-, C₁-C₈-alkoxy- or halogen-monosubstituted, disubstituted, -trisubstituted, -tetrasubstituted or -pentasubstituted C₃-C₁₂-cycloalkyl, aryl, C₇-C₂₀-aralkyl, C₇-C₂₀-alkylaryl, -[(CH₂)₂-O]n-R⁴, -{[CH(CH₃)CH₂]-O}ₘ-R⁵, or R¹ and R² are together an uninterrupted or oxygen-, nitrogen- or sulfur-interrupted C₃-C₁₂-alkylene diradical chain,
R⁴ and R⁵ are each C₁-C₁₂-alkyl, C₃-C₁₂-cycloalkyl, hydroxyl, -COR⁶ or -CH₂-COOR⁷, C₃-C₁₂-cycloalkyl, aryl, C₇-C₂₀-aralkyl or C₇-C₂₀-alkylaryl,
R⁶ and R⁷ are each C₁-C₁₂-alkyl or C₃-C₁₂-cycloalkyl, and
m and n are each from 1 to 40,
from amines of the general formula II 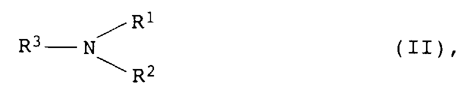 where R¹, R² and R³ are each as defined above, using hydrogen/oxygen mixtures at temperatures from (-5) to 90°C and pressures from 1 to 100 bar in the presence of oxidation catalysts, which comprises using as oxidation catalysts those of group VIII of the periodic table on titanium silicates, vanadium silicates or their mixtures with a zeolite structure.

2. A process for preparing amine oxides I as claimed in claim 1, wherein the oxidation catalysts include from 0.01 to 20% by weight of the elements of group VIII of the periodic table.

3. A process for preparing amine oxides I as claimed in claim 1 or 2, wherein the oxidation catalysts include as elements of group VIII of the periodic table ruthenium, rhodium, palladium, osmium, iridium, platinum or mixtures thereof.

4. A process for preparing amine oxides I as claimed in any of claims 1 to 3, wherein the oxidation catalysts additionally include one or more elements selected from the group consist; ing of rhenium, silver and gold.

5. A process for preparing amine oxides I as claimed in any of claims 1 to 3, wherein the oxidation catalysts contain from 0.01 to 20% by weight of the elements of group VIII of the periodic table and from 99.99 to 80% by weight of titanium silicates, vanadium silicates or their mixtures with a zeolite structure.

6. A process for preparing amine oxides I as claimed in any of claims 1 to 5, wherein the elements of group VIII of the periodic table are present in at least two different bonding energy states.

7. A process for preparing amine oxides I as claimed in any of claims 1 to 6, wherein the oxidation catalysts include as elements of group VIII of the periodic table palladium which is present in two or three different bonding energy states.

8. A process for preparing amine oxides I as claimed in any of claims 1 to 7, wherein the molar ratio of silicon to titanium, vanadium or mixtures thereof in the oxidation catalyst is from 100:1 to 5:1.

9. A process for preparing amine oxides I as claimed in any of claims 1 to 8, wherein R¹, R² and R³ are each C₁-C₄-alkyl, R¹ and R² are together -(CH₂)₂-O-(CH₂)₂- or -(CH₂)₂-NR¹-(CH₂)₂-, and R³ is additionally n-decyl, n-dodecyl, n-tetradecyl, n-hexadecyl or n-octadecyl.

10. A process for preparing amine oxides I as claimed in any of claims 1 to 9, wherein R¹ and R² are together -(CH₂)₂-O-(CH₂)₂- and R³ is methyl.

## Revendications

1. Procédé de préparation d'oxydes d'amine de formule générale I 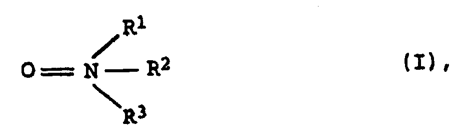 dans laquelle
R¹,R²,R³ représentent des groupements alkyle en C₁-C₃₀, cycloalkyle en C₃-C₁₂, hydroxyalkyle en C₁-C₃₀, aminoalkyle en C₁-C₃₀, alcoxyalkyle en C₂-C₃₀, aryle, aralkyle en C₇-C₂₀, alkylaryle en C₇-C₂₀, des groupements cycloalkyle en C₃-C₁₂, aryle, aralkyle en C₇-C₂₀, alkylaryle en C₇-C₂₀, -[(CH₂)₂-O]ₙ-R⁴, -{[CH(CH₃)CH₂]-O}ₘ-R⁵ substitués une à cinq fois par des groupements alkyle en C₁-C₈, alcoxy en C₁-C₈, ou des atomes d'halogène, ou bien R¹ et R² représentent ensemble une chaîne alkylène en C₃-C₁₂, éventuellement interrompue par des atomes d'oxygène, d'azote ou de soufre,
R⁴,R⁵ représentent des groupements alkyle en C₁-C₁₂, cycloalkyle en C₃-C₁₂, hydroxyle, -COR⁶ ou -CH₂-COOR⁷, cycloalkyle en C₃-C₁₂, aryle, aralkyle en C₇-C₂₀, alkylaryle en C₇-C₂₀,
R⁶, R⁷ représentent des groupements alkyle en C₁-C₁₂ ou cycloalkyle en C₃-C₁₂ et
m, n valent de 1 à 40
à partir d'amines de formule générale II 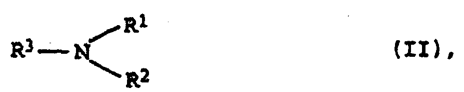 dans laquelle R¹, R² et R³ prennent la signification susmentionnée, avec des mélanges hydrogène/oxygène à des températures de -5 à 90°C et sous des pressions de 1-100 bar, en présence de catalyseurs d'oxydation, caractérisé en ce que l'on utilise, en tant que catalyseurs d'oxydation, des éléments du groupe VIII de la classification périodique des éléments, sur des silicates de titane, des silicates de vanadium ou leurs mélanges ayant une structure de zéolithe.

2. Procédé de préparation d'oxydes d'amine I selon la revendication 1, caractérisé en ce que les catalyseurs d'oxydation contiennent 0,01-20% en poids d'éléments du groupe VIII de la classification périodique des éléments.

3. Procédé de préparation d'oxydes d'amine I selon la revendication 1 ou 2, caractérisé en ce que les catalyseurs d'oxydation contiennent du ruthénium, du rhodium, du palladium, de l'osmium, de l'iridium, du platine ou leurs mélanges, en tant qu'éléments du groupe VIII de la classification périodique des éléments.

4. Procédé de préparation d'oxydes d'amine I selon l'une quelconque des revendications 1 à 3, caractérisé en ce que les catalyseurs d'oxydation contiennent en outre un ou plusieurs éléments choisi dans le groupe constitué par le rhénium, l'argent et l'or.

5. Procédé de préparation d'oxydes d'amine I selon l'une quelconque des revendications 1 à 3, caractérisé en ce que les catalyseurs d'oxydation sont constitués de 0,01-20% en poids d'éléments du groupe VIII de la classification périodique des éléments et de 99,99-80% en poids de silicates de titane, de silicates de vanadium ou de leurs mélanges ayant une structure de zéolithe.

6. Procédé de préparation d'oxydes d'amine I selon l'une quelconque des revendications 1 à 5, caractérisé en ce que les éléments du groupe VIII de la classification périodique des éléments se trouvent dans au moins deux niveaux d'énergie de liaison différents.

7. Procédé de préparation d'oxydes d'amine I selon l'une quelconque des revendications 1 à 6, caractérisé en ce que les catalyseurs d'oxydation contiennent, en tant qu'élément du groupe VIII de la classification périodique des éléments, du palladium se trouvant dans deux ou trois niveaux d'énergie de liaison différents.

8. Procédé de préparation d'oxydes d'amine I selon l'une quelconque des revendications 1 à 7, caractérisé en ce que le rapport molaire du silicium au titane, au vanadium ou à leurs mélanges s'élève de 100: 1 à 5 : 1 dans le catalyseur d'oxydation.

9. Procédé de préparation d'oxydes d'amine I selon l'une quelconque des revendications 1 à 8, caractérisé en ce que R¹, R², R³ représentent un groupement alkyle en C₁-C₄, R¹ et R² représentent ensemble un groupement -(CH₂)₂-O-(CH₂)₂- ou -(CH₂)₂-NR¹-(CH₂)₂-, R³ représente en outre un groupement n-décyle, n-docédyle, n-tétradécyle, n-hexadécyle et n-octadécyle.

10. Procédé de préparation d'oxydes d'amine I selon l'une quelconque des revendications 1 à 9, caractérisé en ce que R¹ et R² représentent ensemble un groupement -(CH₂)₂-O-(CH₂)₂- et R³ représente un groupement méthyle.
